# EUROPÄISCHE PATENTSCHRIFT

(11) **EP 0 598 365 B1**
(45) Veröffentlichungstag und Bekanntmachung des Hinweises auf die Patenterteilung: **11.09.1996**
(21) Anmeldenummer: 93118411.3
(22) Anmeldetag: 13.11.1993
(51) Int. Cl.: A61K 31/20, A61K 33/06

(54) **Mittel zur Behandlung des atopischen Ekzems und anderen entzündlichen Hautkrankheiten**
Agent for the treatment of an atopic eczema and other inflammatory skin diseases
Agent pour le traitement d'un eczème atopique et d'autres maladies inflammatoires de la peau

(30) Priorität: 18.11.1992 DE 4238869
(43) Veröffentlichungstag der Anmeldung: 25.05.1994
(73) Patentinhaber: WOGEPHARM GmbH, D-50354 Hürth (DE)
(72) Erfinder: Diezel, W., Prof. Dr., D-10117 Berlin (DE)
(74) Vertreter: Werner, Hans-Karsten, Dr.Dipl.-Chem.

(56) Entgegenhaltungen:
- EP-A- 0 391 218
- EP-A- 0 432 700
- EP-A- 0 439 640
- WO-A-90/14824
- US-A- 4 885 157
- Dialog Information Services, File 73, Embase 1974-1994, Accession Number: 5119554: J. Holistic Med. vol. 3/2, 1981, pages 118-139, D.F. Horrobin: The importance of gamma-linolenic
- Dialog Information Services, File 73, Embase 1974-1994, Accession Number: 5119554, J. Holistic Med. vol. 3/2, 1981, pages 118-139; D.F. Horrobin: "The importance of gamma-linolenic acid and prostaglandin E1 in human nutrition and medicine"

## Beschreibung

Die Erfindung betrifft ein Mittel zur Behandlung des atopischen Ekzems und anderen entzündlichen Hautkrankheiten. Die Anwendungsgebiete der Erfindung erstrecken sich auf das Gesundheitswesen, die kosmetische und die pharmazeutische Industrie.

Bei folgenden entzündlichen Hautkrankheiten wird als Folge der Bildung des Entzündungsmediators Leukotrien B₄ (LTB₄) in der Haut der Betroffenen die Krankheit ausgelöst bzw. unterhalten: Atopisches Ekzem (B.M. Czarnetzki et al.: Clin. Exp. Immunol. 54 (1983) 486; K. Fogh et al.: J. Allergy Clin. Immunol. 83 (1989) 68; T. Ruzicka et al: J. Invest. Dermatol. 86 (1986) 105), Psoriasis (S. Brain et al.: J. Invest. Dermatol. 83 (1984) 70; K. Fogh et al.: Arch. Dermatol. Res. 278 (1986) 173) und allergisches Kontaktekzem (R.M. Barr et al.: Br. J. Dermatol. 111 (1984) 23; H. Bisgaard et al.: Allergy 40 (1985) 417.

Dies impliziert, daß durch eine Hemmung der Bildung von LTB₄ in der Haut bzw. durch eine Inaktivierung von LTB₄ in der Haut die o.g. entzündlichen Hautkrankheiten behandelt werden können. Tatsächlich ist dies der Fall: Glukokortikosteroide und andere Phospholipase A2-Hemmer bzw. 5-Lipoxygenase-Hemmer sind therapiewirksam beim atopischen Ekzem, bei Psoriasis und auch beim kontaktallergischen Ekzem (Übersichten: B.C. Bastian et al.: Hautarzt 42 (1991) 417; R.D.R. Camp u. F.F. Derm: J. Invest. Dermatol. 92 (1989) 789; B.R. Allen u. S.M. Littlewood: Br. Med. J. 285 (1982) 1241).

Die genannten entzündungshemmenden therapeutischen Mittel haben bei äußerlicher Anwendung folgende Nachteile:
- Glukokortikosteroide: Hautatrophie, Hautalterung, Aufnahme durch die Haut und systemische Nebenwirkungen.
- Lipoxygenase-Hemmer: Toxische Nebenwirkungen bei äußerlicher Anwendung.

Es ist bekannt, daß bestimmte Fettsäuren die Bildung von LTB₄ hemmen:
- Gamma-Linolensäure (18:3n-6): Aus Gamma-Linolensäure entsteht 15-Hydroxy-dihomo-gamma-linolensäure. Dieses Produkt des Enzyms 15-Lipoxygenase hemmt das Enzym 5-Lipoxygenase, welche LTB₄ synthetisiert (C.C. Miller et al.: Prostaglandins 35 (1988) 917.
- Eicosapentaensäure (20:5n-6): Aus Eicosapentaensäure entsteht Leukotrien B5, daß die Bildung von LTB₄ hemmt (Lee et al.: Advances in Immunology 39 (1986) 145; Terano et al.: Prostaglandins 27 (1984) 217).

Die o.g. Fettsäuren sind oftmals in diätetischen Mitteln enthalten, um die Therapie entzündlicher Hautkrankheiten zu unterstützen (C.C. Miller u. V.A. Ziboh: Biochem. Biophy. Res. Commun. 154 (1988) 967; S. Wright u. J.L. Burton: Lancet 2 (1982) 1120).

Die DE-A-33 25 130 beschreibt pharmazeutische Zusammensetzungen zur Anwendung für die Haut auf der Basis von essentiellen Fettsäuren.

Die EP-A 0 439 640 beschreibt ein Mittel zur Therapie entzündlicher Hautkrankheiten bzw. zur Abschwächung allergischer, entzündlicher Reaktionen der Haut durch äußerliche Anwendung von wasserlöslichen Magnesiumsalzen, insbesondere Magnesiumchlorid.

Die US-A-4,885,157 beschreibt Produkte, die zumindest zu 80 aus lebenden Hefezellen, Seien, Karotin, RNA, DNA, Wasser und Albumin bestehen. Gemäß Beispiel 4 enthält diese Zubereitung zusätzlich auf neun Gewichtsteile einen Gewichtsteil einer weiteren ergänzenden Mischung. Diese Mischung soll 2,1 mg Calcium, 60 mg Magnesium, 18 mg Niazin und 5,7 mg Kupfer enthalten. Es fehlen Angaben über die weiteren Komponenten und in welcher Form diese Metalle vorliegen sollen.

Die EP-A-0 391 218 beschreibt orale Zubereitungen, die nicht zur Therapie sondern zur Prophylaxe der Atopie eingesetzt werden sollen. Derartigen Zubereitungen können Calcium, Zink und Eisensalze einen zusätzlichen Effekt geben, da es sich um die frühzeitige und richtige Ausbildung des Immunsystems handelt, nicht jedoch um die Behandlung des atopischen Ekzems und anderer entzündliche Hautkrankheiten, das heißt bereits manifester Erkrankungen.

Der Erfindung liegt die Aufgabe zugrunde, der medizinischen Praxis neue Mittel in die Hand zu geben, die sich für die Therapie entzündlicher Hautkrankheiten, insbesondere des atopischen Ekzems, eignen.

Die Lösung dieser Aufgabe erfolgt durch Mittel zur Behandlung des atopischen Ekzems und anderen entzündlichen Hautkrankheiten bestehend aus einer äußerlich anzuwendenden Zubereitung enthaltend mindestens 0,5 Gew.-% Gamma-Linolensäure und/oder Eicosapentaensäure sowie zusätzlich 1 bis 30 Gew.-% wasserlösliche Magnesiumsalze.

Es wurde nämlich gefunden, daß die Gamma-Linolensäure und/oder Eicosapentaensäure zusammen mit Magnesiumsalzen besonders gute und nachhaltige Wirkungen zeigen. Die erfindungsgemäßen Mittel sind somit wirksamer als die beiden Einzelkomponenten.

Die Konzentration der Fettsäuren liegt in einem Bereich von 0,5 bis 50,0 Gew.-%, wobei die Fettsäuren in Trägermaterialien eingearbeitet werden, die auf die Haut aufgetragen werden können.

Die Konzentration des Magnesiums liegt in einem Bereich von 1,0 bis 30,0 Gew.-% im o.g. Trägermaterial.

Das Wesen der Erfindung wird anhand einiger Ausführungsbeispiele näher beschrieben.

Dabei wird als tierexperimentelles Modell das Modell des "Crotonöl-induzierten entzündlichen Ödems" verwendet. Es ist bekannt, daß die Entzündung bei diesem Modell primär als Folge der Wirkung von LTB₄ zustande kommt (C.B. Archer et al.: Prostaglandins 33 (1987) 799; S.E. Dahlen et al.: Proc. Natl. Acad. Sci (USA) 78 (1981) 3887; M. Katori et al.: Prostaglandins 28 (1984) 617).

### Beispiel 1:

Hemmung des Crotonöl-induzierten entzündlichen Ödems durch Gamma-Linolensäure, Nachtkerzenöl, Borretschöl, Rinderklauenöl und Erdnußöl allein und in Kombination mit MgCl₂ (Methodik: R.J. Dorfman: Br. J. Dermatol. 82 (Suppl. 6) (1970) 45).

Die Untersuchen wurden an AB/Bln.-Mäusen (männliche Tiere, Gewicht 18 - 25 g) durchgeführt. Jede Tiergruppe bestand aus 8 Tieren.

Zur Auslösung der Entzündung (des Crotonöl-Ödems) wurde ein Ohr (rechtes äußeres und inneres Ohrblatt jedes Tieres mit je 10 µl Crotonöl (1%ig, gelöst in Aceton) behandelt. Auf das linke Ohr wurden gleichartig 10 µl Aceton aufgetragen. Eine Stunde später wurden auf das rechte Ohr 10 µl der in Tabelle 1 vorgestellten Fettsäuren allein bzw. inkorporiert in ein Trägermedium zusammen mit MgCl₂ aufgetragen. Dabei wurde das rechte Ohr mit 2,5 µg dieser Kombination bzw. mit 2,5 µg der Hydrocortison Wolff^{R} 0,5 Creme behandelt (Nr. 2 und Nr. 3 in Tabelle 1).

Fünf Stunden später wurden die Tiere durch zervikale Dislokation getötet, und es wurde aus den Ohren jedes Tieres ein Gewebestück von 8 mm ausgestanzt. Beide Gewebestücke wurden gewogen, und es wurde das Gewicht des Gewebestückes "linkes Ohr" vom Gewebestück "rechtes Ohr" subtrahiert. Die Differenz des Gewichtes entspricht dem Gewicht des "entzündlichen Ödems" (angegeben in mg in Tabelle 1).

### Beispiel 2:

Entzündungshemmende Wirkung von Nachtkerzenöl kombiniert mit MgCl₂ in einer Salbengrundlage (Nr. 3 von Tabelle 1) bei einem Patienten mit atopischem Ekzem. Nach 10tägiger äußerlicher Behandlung von Patienten mit einem atopischen Ekzem resultiert eine beschleunigte Abheilung dieser entzündlichen Dermatose (A). Im Vergleich dazu hemmte die alleinige Salbengrundlage die Entzündung nicht (B).

Die reine Gamma-Linolensäure bzw. Öle mit einem natürlichen Anteil an Gamma-Linolensäure bzw Eicosapentaensäure sind in Konzentrationen von 0,5 % bis 50,0 % in allen Grundlagen, die auf die Haut aufgetragen werden können, diesbezüglich antientzündlich wirksam.

Diesen Zubereitungen wird 1 bis 30 Gew.-% Magnesiumchlorid beigemischt. Diese Zubereitungen sind auch im Gemisch stabil. Eine weitere, gut geeignete Salbengrundlage ist Alcoholum emulsificans non ionogenicum. Prinzipiell sind aber alle üblichen Grundlagen für Salben, Cremes, Lotionen und Gele geeignet, die auch sonst für äußerliche Anwendungen zum Einsatz kommen.

Aus der Tabelle 2 geht hervor, daß die beiden Einzelkomponenten Magnesiumchlorid und Nachtkerzenöl für sich alleine in der gleichen Salbengrundlage jeweils nur eine prozentuale Hemmung des entzündlichen Ödems von 37 bzw. 30,6 % zeigen. Die erfindungsgemäße Kombination dieser beiden Wirkstoffe zeigt aber eine prozentuale Hemmung von 58 % und erzielt damit erstmals eine vergleichbar gute Wirkung wie Hydrocortison. Es war nicht vorhersehbar, daß es möglich ist, mit der Kombination der zwei natürlichen und ungefährlichen Wirkstoffe bei einer so weit verbreiteten und dennoch sehr unangenehmen und ständig behandlungsbedürftigen Krankheit, wie die atopischen Ekzeme, zu so guten Ergebnissen zu kommen. Offensichtlich addieren sich bei dem erfindungsgemäßen Mittel nicht nur die Wirkungen der beiden Komponenten, sondern führen zwei unterschiedliche Wirkmechanismen zu den Therapieerfolgen.

**Tabelle 1**

| Hemmung des Crotonöl-induzierten entzündlichen Ödems durch Fettsäuren bzw. in Kombination mit MgCl₂ in einem Trägermedium | | | | |
|---|---|---|---|---|
| Nr. | Tiergruppe¹⁾ | mg (x) | Entzündliches Ödem (prozentuale Hemmung) | < p²⁾ |
| 1 | Kontrolle (alleinige Crotonöl-Behandlung) | 17,09 | 0 | |
| 2 | Hydrocortison Wolff^{R} 0,5 | 7,00 | 59,0 | 0,01 |
| 3 | Nachtkerzenöl 10,0 | 7,19 | 58,0 | 0,01 |
| | MgCl₂ | | | |
| | Salbengrundlage ad 100,0³⁾ | | | |
| 4 | Gamma-Linolensäure | 7,33 | 57,1 | 0,01 |
| 5 | Borretschöl | 7,50 | 56,0 | 0,01 |
| 6 | Rinderklauenöl | 7,51 | 56,0 | 0,01 |
| 7 | Erdnußöl | 7,62 | 55,4 | 0,01 |
| 8 | Nachtkerzenöl | 8,21 | 52,0 | 0,05 |
| 9 | Olivenöl | 13,19 | 22,8 | N.S. |
| 10 | Leinöl | 14,06 | 17,7 | N.S. |

| | | | | |
|---|---|---|---|---|
| ¹⁾ Jede Tiergruppe bestand aus 8 AB/Bln.-Mäusen | | | | |
| ²⁾ Wilcoxon-Test: Statistische Differenz zur Kontrolle. N.S. = nicht signifikanter Unterschied | | | | |
| ³⁾ Salbengrundlage: Unguentum Alcoholum Lanae aquosum (DAB7) | | | | |

**Tabelle 2**

| Hemmung des Crotonöl-induzierten entzündlichen Ödems allein durch Nachtkerzenöl bzw. allein durch MgCl₂ und in dieser Kombination in einem Trägermedium | | |
|---|---|---|
| Nr. | Tiergruppe¹⁾ | Entzündliches Ödem [prozentuale Hemmung]²⁾ |
| 1. | Kontrolle | 0 |
| 2. | MgCl₂ 5,0 | 37,0 |
| | Salbengrundlage ad 100,0³⁾ | |
| 3. | Nachtkerzenöl 10,0 | 30,6 |
| | Salbengrundlage ad 100,0 | |
| 4. | Nachtkerzenöl 10,0 | 58,0 |
| | MgCl₂ 5,0 | |
| | Salbengrundlage ad 100,0 | |

| | | |
|---|---|---|
| ¹⁾ Jede Tiergruppe bestand aus 8 AB/Bln.-Mäusen | | |
| ²⁾ Wilcoxon-Test: Statistische Differenz Nr. 2/4: 0,05 Nr. 3/4: 0,01 | | |
| ³⁾ Salbengrundlage: Unguentum Alcoholum Lanae aquosum (DAB7) | | |

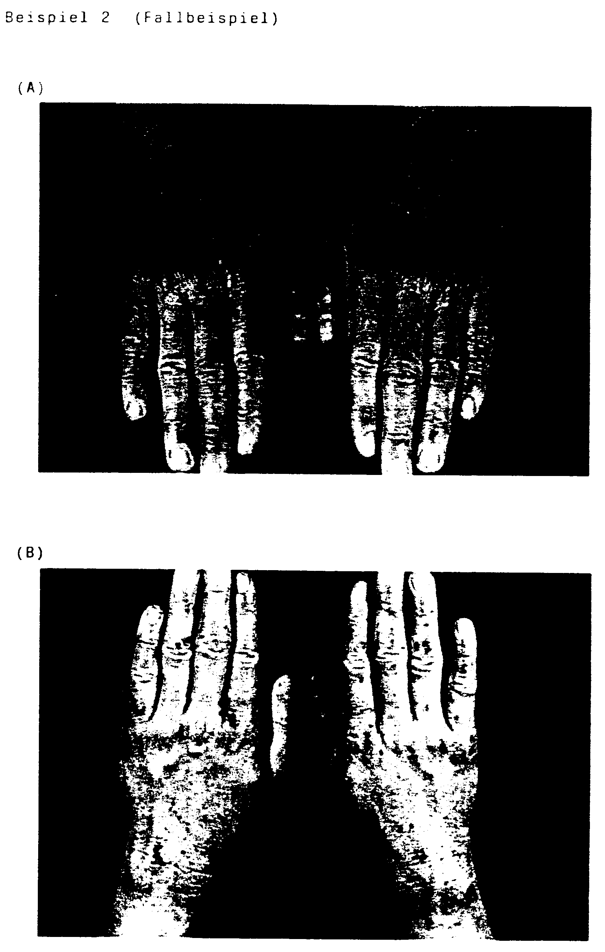

## Patentansprüche

1. Mittel zur Behandlung des atopischen Ekzems und anderen entzündlichen Hautkrankheiten bestehend aus einer äußerlich anzuwendenden Zubereitung enthaltend mindestens 0,5 Gew.-% Gamma-Linolensäure und/oder Eicosapentaensäure sowie zusätzlich 1 bis 30 Gew.-% wasserlösliche Magnesiumsalze.

2. Mittel gemäß Anspruch 1, dadurch gekennzeichnet, daß die Säuren zugesetzt werden in Form von Nachtkerzenöl, Borretschöl, Rinderklauenöl, Erdnussöl oder Gemische derselben.

3. Mittel gemäß Anspruch 1 oder 2, dadurch gekennzeichnet, daß es Magnesiumchlorid enthält.

4. Verwendung von mindestens 0,5 Gew.-% Gamma-Linolensäure und/oder Eicosapentaensäure zusammen mit 1 bis 30 Gew.-% wasserlöslichen Magnesiumsalzen zur Herstellung von äußerlich anwendbaren Zubereitungen zur Behandlung des atopischen Ekzems und anderen entzündlichen Hautkrankheiten.

5. Verwendung gemäß Anspruch 4, dadurch gekennzeichnet, daß Nachtkerzenöl, Borretschöl, Rinderklauenöl, Erdnussöl oder Gemische derselben eingesetzt werden.

6. Verwendung gemäß Anspruch 4 oder 5, dadurch gekennzeichnet, daß als wasserlösliches Magnesiumsalz Magnesiumchlorid eingesetzt wird.

## Claims

1. An agent for the treatment of atopic eczema and other inflammatory skin diseases consisting of a formulation for external application containing at least 0.5% by weight of gamma-linolenic acid and/or eicosapentaenic acid and in addition from 1 to 30% by weight of water-soluble magnesium salts.

2. The agent according to claim 1, characterized in that said acids are added in the form of evening primrose oil, borage oil, neatsfoot oil, peanut oil or mixtures thereof.

3. The agent according to claim 1 or 2, characterized by containing magnesium chloride.

4. The use of at least 0.5% by weight of gamma-linolenic acid and/or eicosapentaenic acid together with from 1 to 30% by weight of water-soluble magnesium salts for the preparation of formulations for external application for the treatment of atopic eczema and other inflammatory skin diseases.

5. The use according to claim 4, characterized in that evening primrose oil, borage oil, neatsfoot oil, peanut oil or mixtures thereof are employed.

6. The use according to claim 4 or 5, characterized in that magnesium chloride is employed as said water-soluble magnesium salt.

## Revendications

1. Agent pour le traitement de l'eczéma atopique et d'autres maladies inflammatoires de la peau, formé d'une préparation à usage externe contenant au moins 0,5 % en poids d'acide gamma-linolénique et/ou d'acide eicosapentanoïque, ainsi, qu'en outre, 1 à 30 % en poids de sels de magnésium solubles dans l'eau.

2. Agent selon la revendication 1, caractérisé en ce qu'on ajoute les acides sous la forme d'huile d'onagre, d'huile de bourrache, d'huile d'ongles de bovins, d'huile d'arachide ou de mélanges de celles-ci.

3. Agent selon la revendication 1 ou 2, caractérisé en ce qu'il contient du chlorure de magnésium.

4. Utilisation d'au moins 0,5 % en poids d'acide gamma-linolénique et/ou d'acide eicosapentanoïque, conjointement avec 1 à 30 % en poids de sels de magnésium solubles dans l'eau, pour l'obtention de préparations à usage externe pour le traitement de l'eczéma atopique et d'autres maladies inflammatoires de la peau.

5. Utilisation selon la revendication 4, caractérisée en ce qu'on utilise de l'huile d'onagre, de l'huile de bourrache, de l'huile d'ongles de bovins, de l'huile d'arachide ou des mélanges de celles-ci.

6. Utilisation selon la revendication 4 ou 5, caractérisée en ce qu'on utilise du chlorure de magnésium comme sel de magnésium soluble dans l'eau.
